# EUROPÄISCHE PATENTSCHRIFT

(11) **EP 0 757 575 B1**
(45) Veröffentlichungstag und Bekanntmachung des Hinweises auf die Patenterteilung: **17.09.2003**
(21) Anmeldenummer: 96903925.4
(22) Anmeldetag: 26.02.1996
(51) Int. Cl.: A61N 1/05

(54) **ELEKTRODENKATHETER ZUR KARTIERENDEN ERFASSUNG VON REIZLEITUNGSSTRUKTUREN**
ELECTRODE CATHETER FOR MAPPING CARDIONECTOR STRUCTURES
CATHETER A ELECTRODE POUR CARTOGRAPHIE DE STRUCTURES DU SYSTEME DE CONDUCTION

(30) Priorität: 24.02.1995 DE 19507929
(43) Veröffentlichungstag der Anmeldung: 12.02.1997
(73) Patentinhaber: BIOTRONIK Mess- und Therapiegeräte GmbH & Co Ingenieurbüro Berlin, 12359 Berlin (DE)
(72) Erfinder: BOLZ, Armin, D-91058 Erlangen (DE); SCHALDACH, Max, D-91054 Erlangen (DE); WETZIG, Thomas, D-91088 Bubenreuth (DE); ZRENNER, Bernhard, D-84072 Reichertshausen (DE)
(74) Vertreter: Christiansen, Henning
(86) Internationale Anmeldenummer: DE9600353
(87) Internationale Veröffentlichungsnummer: WO96025974

(56) Entgegenhaltungen:
- EP-A- 0 237 316
- EP-A- 0 499 491
- EP-A- 0 500 215
- EP-A- 0 664 990
- WO-A-92/21278
- WO-A-94/00050
- WO-A-94/06507
- WO-A-94/21168
- WO-A-95/17222

## Beschreibung

Die Erfindung betrifft einen Elektrodenkatheter der im Oberbegriff des Anspruchs 1 angegebenen Art.

Der räumlich kartierende Erfassung der Reizleitungsstrukturen im Herzen (anglisierend kurz als "Mapping" bezeichnet) sowie ggfs. gezielten thermischen Zerstörung ("Ablation") von Abschnitten dieser Reizleitungsstrukturen durch lokalisierten Eintrag von HF-Energie in Auswertung des Kartierungsergebnisses ist in den letzten Jahren sowohl im Rahmen von Grundlagen- als auch bereits von klinischen Untersuchungen zur Therapie spezieller Tachykardien - vornehmlich der sogenannten "reentrant"- Tachykardien - und atrialer sowie ventrikulärer Fibrillationen großes Augenmerk geschenkt worden. Die hierbei eingesetzten Hilfsmittel und Techniken, vor allem spezielle endokardiale Mapping- und Ablationskatheter, haben dabei eine Vielzahl wesentlicher Weiterentwicklungen erfahren.

Mit Hilfe eines Mapping-Katheters wird im Rahmen einer Untersuchung - jeweils mittels auf einem Träger in exakt vorgegebener Lage angeordneter Elektroden - eine Vielzahl definiert lokaler Impedanzmessungen am Herzgewebe vorgenommen. Im Ergebnis dieser Messungen erhält man eine "Karte" von (insbesondere durch Aussendung eines Stimulus an der Katheterspitze stimulierten) Herzmuskel-Entladungspotentialen und kann anhand dieser kardiale Läsionen und arrythmie-erzeugende Zentren bzw. Knoten lokalisieren. Wurde ein entsprechendes Zentrum lokalisiert, können Ablationselektroden des Katheters dort positioniert werden, um durch einen HF- oder Gleichspannungs-stromstoß eine lokale Gewebskoagulation hervorzurufen und das arrhythmieerzeugende Zentrum zu zerstören.

Die thermische Ablation stellt einen minimal-invasiven Eingriff dar und ist aufgrund wesentlich verringerter Gefahren für den Patienten einem dem gleichen Ziel dienenden chirurgischen Eingriff am offenen Herzen entschieden vorzuziehen.

Ein noch relativ einfach aufgebauter steuerbarer Mapping-und Ablationskatheter ist etwa in WO-A-94/00050 beschrieben, wo sich auch eine komprimierte Beschreibung des Einsatzes und der dazu benötigten Steuerung findet, die grundsätzlich auch für die nachfolgend beschriebenen Katheter gemäß Ausführungsformen der Erfindung gilt. Der in dieser Druckschrift beschriebene Katheter weist eine Mehrzahl von axial beabstandeten, einzeln mit einer Erfassungs- und Steuerschaltung am proximalen Ende verbundenen Ringelektroden auf einem langgestreckten isolierenden Träger auf. Zum Elektrodenmaterial findet sich hier keine Aussage.

In WO-A-95 09561 ist ein ähnlicher multipolarer Katheter beschrieben, bei dem Goldelektroden eingesetzt werden.

Bei einer weiteren ähnlichen Anordnung nach WO-A-94 21170 wird Platin/Iridium als Elektrodenmaterial vorgeschlagen.

Ein ebenfalls ähnlicher Ablstionskatheter mit resistiven Mapping-Elektroden, für die als Materialien Edelstahl, Gold, Silber-Silberchlorid oder Platin vorgeschlagen werden, ist in WO-A-94/00049 beschrieben.

In der WO 92/21278 ist ein Katheter offenbart, der einerseits zur Erfassung eines monophasischen Aktionspotentials und andererseits zur Ablation geeignet ist. Mit einem besonderen elektronischen Filtersystem sollen die während der Ablation auftretenden Störungen bei der Erfassung eliminiert werden.

Die WO 94/06507 zeigt ein implantierbares Defibrillatorsystem mit einer intrakardialen oder subkutanen Defibrillationselektrode, wobei die Defibrillationselektrode mit einer porösen Oberflächenbeschichtung versehen ist.

Aus der WO 94/00050 ist ein Positioniersystem für einen Ablationskatheter bekannt, bei dem anhand eines von mehreren Elektroden erzeugten und erfassten Differenzsignals die Position einer Tippelektrode bestimmt wird.

In der EP 0 500 215 ist ein Verfahren und ein Gerät zur Erfassung von Tachyarrhytmien beschrieben. Der eingesetzte Katheter kann auch zur Ablation genutzt werden.

Aus der WO 94/21168 ist ein Katheter zur kartierenden Erfassung und Ablation bekannt. Die Sensorelektroden können auch für die Ablation genutzt werden.

Ein endokardiales Mapping- und Ablationssystem ist ferner in der EP 0 499 491 beschrieben. Das System zeigt Elektroden, die gleichzeitig als Erfassungs- und Ablationselektroden ausgebildet sind. Die Elektroden umfassen eine dünne Kupferschicht, die aufgesputtert wird. Weitere Schichten aus Nickel oder Gold können folgen.

Weiterhin ist aus der EP 0 237 316 eine Herzschrittmachereinheit mit einer Elektrodenleitung bekannt, die eine Elektrode mit einer Außenbeschichtung aus Iridiumoxid umfasst.

Schlussendlich beschreibt die EP 0 664 990 einen Mapping- und Ablationskatheter, bei dem die distale Elektrode als Erfassungs- und Ablationselektrode ausgebildet ist. Die Elektrode besteht aus einem elektrisch leitfähigem Träger, wie beispielsweise Kupfer oder Silber. Sie weist ferner die Oberfläche bedeckende Beschichtung aus Gold oder eine Goldlegierung auf, die durch Vakuumabscheidung erzeugt wird.

Der Erfindung liegt die grundsätzliche Aufgabe zugrunde, Mittel zur verbesserten kartierenden Erfassung und - darauf beruhend - lokalisierten Zerstörung von Reizleitungsstrukturen im Herzgewebe anzugeben.

Diese Aufgabe wird durch einen Elektrodenkatheter mit den Merkmalen des Anspruchs 1 gelöst.

Im Rahmen entsprechender Untersuchungen haben die Erfinder festgestellt, daß die Aussagekraft des Mapping und damit die therapeutischen Ergebnisse der auf den Ergebnissen des Mapping beruhenden Ablation durch eine genaue Erfassung der Kurvenform bestimmter Herzaktionspotentiale, speziell des (atrialen) monophasischen Aktionspotentials MAP, signifikant verbessert werden können.

weiter haben sie festgestellt, daß hierfür in vorteilhafter Weise Elektroden mit sehr niedriger Elektrodenimpedanz und - speziell für evozierte Myokardpotentiale, wie etwa das stimulierte MAP - geringer Polarisierbarkeit einzusetzen sind. Derartige Elektroden ermöglichen die Erfassung auch sehr niederfrequenter und (bei evozierten Potentialen) dem Stimuluspuls zeitlich relativ eng, d.h. bis zu wenigen zehn Millisekunden, benachbarter Signalanteile und damit eine präzise Erfassung der Gesamt-Signalform.

Als besonders geeignet hat sich zum einen die Ausführung der der Signalerfassung (dem eigentlichen Mapping bzw. "Sensing") dienenden Elektrodenabschnitte mit einer im wesentlichen aus Iridiumoxid ("IrOx") bestehenden elektroaktive Beschichtung erwiesen.

Alternativ hierzu können diese Elektrodenabschnitte eine im wesentlichen aus Iridium bestehende Beschichtung mit fraktaler Oberflächengeometrie aufweisen.

Obgleich bei herkömmlichen Ablationskathetern die Sensing- in der Regel von den Ablationselektroden getrennt sind, ist mindestens eine der Erfassungs-Elektroden zugleich als Ablationselektrode ausgebildet .

Die Ausführung kann dabei dergestalt sein, daß die üblicherweise im Rahmen der Erfassung evozierter Myokardpotentiale der Aussendung der Stimulusimpulse sowie späterhin der Ablation dienende Elektrode am distalen Ende des Katheters (Spitzenelektrode) zugleich mit fraktaler oder elektroaktiver Oberfläche als Erfassungs-Elektrode ausgebildet ist. Es kann aber andererseits auch eine am Umfang des Katheterschaftes angeordnete, üblicherweise als Erfassungs-Elektrode dienende Elektrode gleichzeitig eine zum Eintrag von Ablationsenergie in das Gewebe geeignete Ausführung erhalten.

Die gleichzeitig als Erfassungs- und Ablationselektrode dienende Elektrode (bzw. deren Mehrzahl) weist eine massive metallische Unterlage mit hoher elektrischer und thermischer Leitfähigkeit, beispielweise eine Gold-Nickel-Legierung, auf.

Bei einem bevorzugten Elektrodenkatheter ist mindestens ein Paar bipolar angeschlossener Erfassungs-Elektroden vorgesehen. Spezieller ist jeweils mindestens ein Paar ring- oder halbringförmiger Erfassungs-Elektroden auf einem langgestreckten flexiblen Träger angeordnet; d.h. der Katheter kann - in an sich bereits bekannter Weise (vgl. etwa US-A-5 228 442) - etwa eine Mehrzahl von gegeneinander aufspreizbaren Trägern mit jeweils einem oder mehreren Elektrodenpaaren aufweisen, oder es ist nur ein Träger mit einem oder mehreren Paaren von Erfassungs-Elektroden vorgesehen.

Zur Ausführung der Kartierung ist dem Träger eine - als solche ebenfalls bekannte - Steuerungseinrichtung zur wahlweisen Einstellung einer vorgegebenen gekrümmten Gestalt oder (bevorzugt) einer Mehrzahl vorbestimmter Formen zugeordnet.

Weiterhin kann in einer Ausbildung, die den gezielten Eintrag von Ablationsenergie in defekte Abschnitte der Reizleitungsstruktur mit minimaler Schädigung des umliegenden Gewebes ermöglicht, eine aktive, insbesondere räumlich mindestens einer der Elektroden zugeordnete, Kühleinrichtung vorgesehen sein. Angesichts der geringen Katheterdurchmesser und zur Sicherung eines vernünftigen Betriebsaufwandes sind hierfür bevorzugt elektrische Kühlelemente vorzusehen, beispielsweise Peltierelemente.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
die Figuren 1a bis 1f grafische Darstellungen der Zeitabhängigkeit monophasischer Aktionspotentiale MAP, aufgenommen mittels fraktaler Elektroden in verschiedenen Anordnungen,
Figur 2 eine Detaildarstellung des distalen Endes eines Elektrodenkatheters mit fraktalen Elektroden zur Erfassung der monophasischen Aktionspotentiale MAP nach Fig. 1a bis 1f,
die Figuren 3a bis 3c schematische Darstellungen zur Verdeutlichung der Oberflächenstruktur (im Querschnitt) einer fraktalen Elektrode, wie sie beim erfindungsgernäßen Elektrodenkatheter vorgesehen ist,
die Figuren 4 und 4a eine (vereinfachte) Gesamtansicht sowie eine Querschnittsdarstellung eines multipolaren steuerbaren Mapping-Elektrodenkatheters gemäß einer Ausführungsform der Erfindung,
die Figuren 5, 5a und 5b eine (vereinfachte) Gesamtansicht, eine Querschnittsdarstellung sowie eine (modifizierte) Teilansicht eines in weitem Winkelbereich steuerbaren Mapping-Elektrodenkatheters gemäß einer weiteren Ausführungsform,
die Figuren 6 und 6a eine (vereinfachte) Gesamtansicht sowie eine Querschnittsdarstellung eines Mapping-Elektrodenkatheters gemäß einer weiteren Ausführungsform,
Figur 7 eine schematische Darstellung zur Verdeutlichung des Aufbaus und Anschlusses der Spitzenelektrode eines Mapping- und Ablationskatheters gemäß einer weiteren Ausführungsform und
Figur 8 ein vereinfachtes Blockschaltbild einer Mapping-Anordnung unter Einsatz des erfindungsgemäßen Elektrodenkatheters.

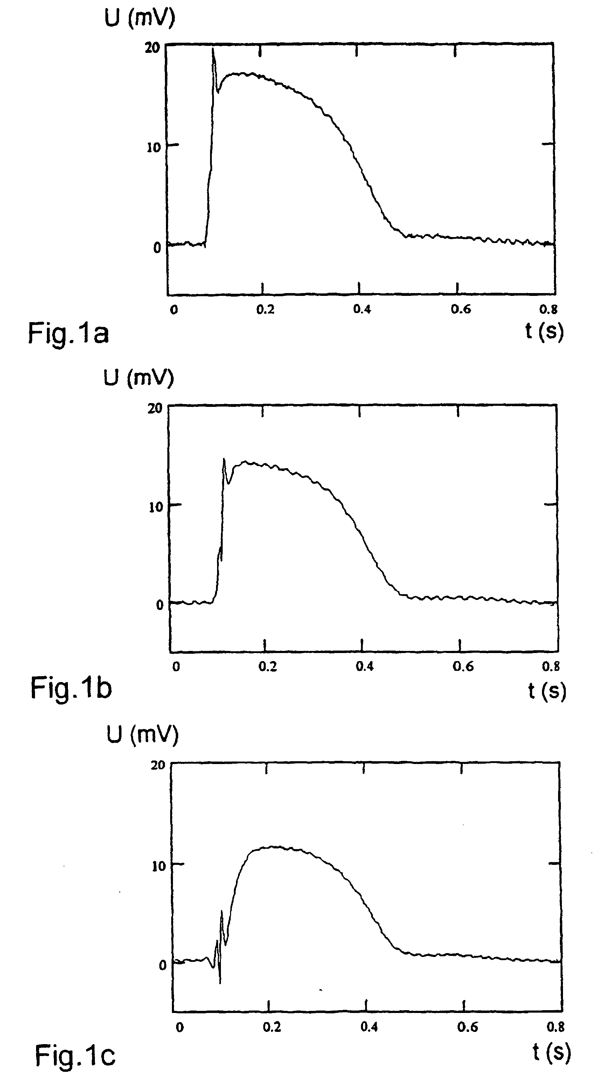

In Fig. 1a bis 1f sind Beispiele für die Zeitabhängigkeit der gemessenen Spannung bei der Erfassung des atrialen monophasischen Aktionspotentials MAP gezeigt, die mittels fraktaler Elektroden in verschiedenen Anordnungen aufgenommen wurden.

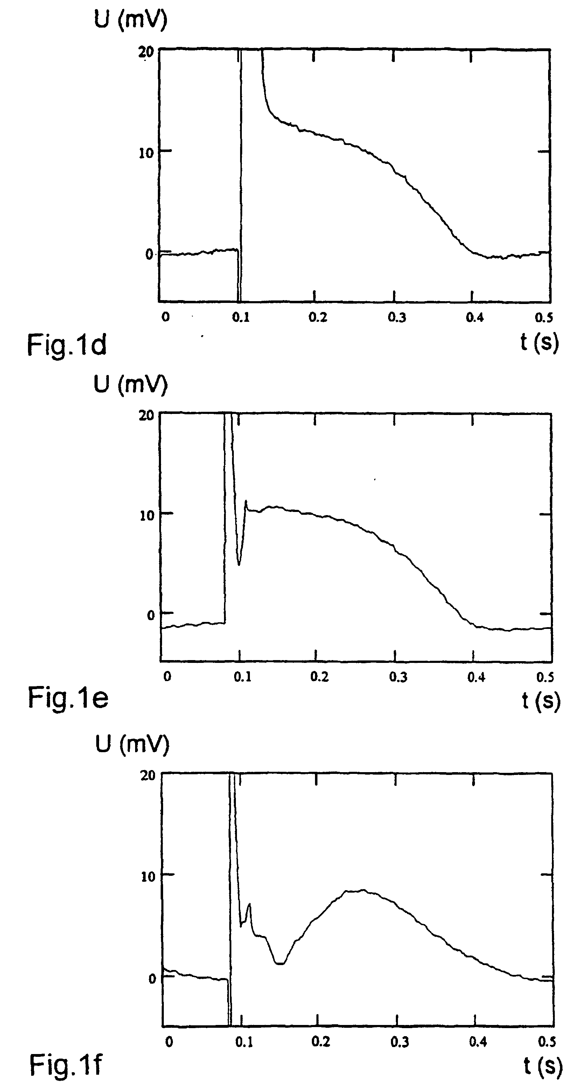

Fig. 1a bis 1c zeigen Verläufe spontan erregter MAP, die bei Elektrodenabständen einer differenten zu einer indifferenten fraktalen Elektrode von 4 mm (Fig. 1a) bzw. 16 mm (Fig. 1b) bzw. unter Anordnung einer indifferenten Elektrode in der vena subclavia (FIg. 1c) aufgenommen wurden, während Fig. 1d bis 1f die Potentialverläufe der durch Stimulation mit 140 l/min, U = 2V über die Elektrodenkombination differente fraktale Elektrode/indifferente fraktale Elektrode in 4 mm Abstand evozierten MAP mit den Fig. 1a bis 1c entsprechenden Erfassungs-Elektrodenanordnungen zeigen. Die charakteristische Morphologie des MAP ist besonders gut in Fig. 1a, 1b bzw. 1e, 1f zu erkennen; bei größeren Elektrodenabständen bzw. - wie in Fig. 1c und 1f - bei Anordnung der indifferenten Elektrode in der Vena subclavia tritt tendentiell eine Änderung der Morphologie hin zu einem intrakardialen EKG (IEKG) bzw. einem herkömmlichen evozierten Potential auf.

Physikalisch stellt das MAP ein Summenpotential dar, das sich aus der Überlagerung von Transmembranpotentialen in Elektrodennähe ergibt und somit physiologisch die Aktivität des autonomen Nervensystems (ANS) reflektiert, wobei in das MAP-Signal aufgrund der Messung im Vorhof sowohl Sympathikus- als auch Parasympathikusaktivität Eingang findet. Es stellt damit eine wertvolle Informationsquelle sowohl für eine frequenzadaptive Herzstimulation als such zur Früherkennung von atrialen (speziell tachykarden) Arrhythmien dar und ist somit nach Erkenntnis der Erfinder als Regelgröße eines ratenadaptiven Schrittmachers ebenso geeignet wie als Meßgröße für die Kartierung (das Mapping) und ggfs. Zerstörung von potentiell arrhythmieerzeugenden Reizleitungsstrukturen im Herzgewebe. Ergebnisse eines Mapping unter Auswertung der MAP-Signale sind auch zur Vorbereitung der optimalen Positionierung von Reizelektroden am Herzmuskel - etwa zur Tachykardieterminierung durch gezielte lokale Stimulation - vorteilhaft nutzbar.

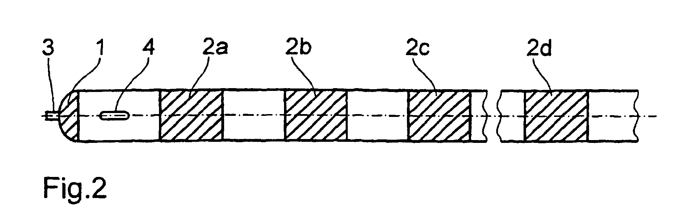

Figur 2 ist eine Detaildarstellung des distalen Endes eines Elektrodenkatheters mit fraktalen Elektroden zur Erfassung der monophasischen Aktionspotentiale MAP nach Fig. 1a bis 1f.

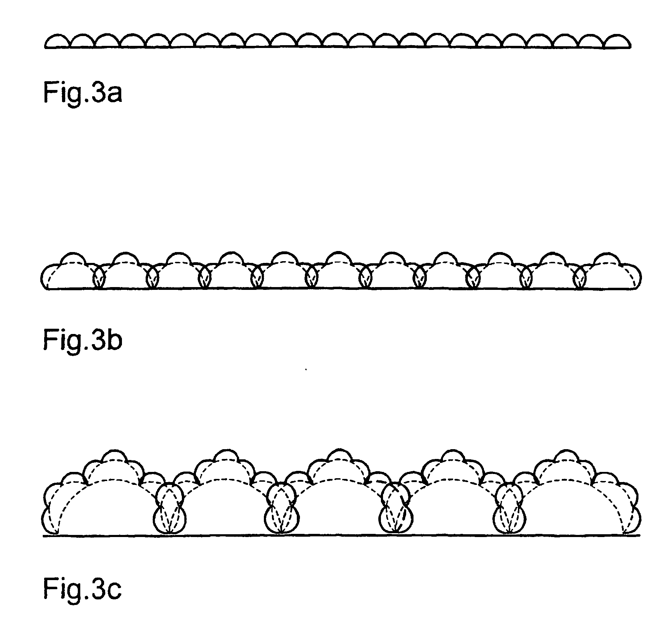

Wie in der Figur zu erkennen, sind eine halbkugelschalenförmige Spitzenelektrode 1 und vier zylinderschalen- bzw. ringförmige Gegenelektroden 2a bis 2d vorgesehen. Der Durchmesser beträgt 1 bis 1,5 mm, die Längen und Abstände der Elektroden 2a bis 2d sind einander gleich und betragen jeweils 3 mm, der Abstand zwischen den Elektroden 1 und 2a beträgt 4 mm. Die Elektroden 1 bis 2d bestehen jeweils aus einem biokompatiblen Träger aus Titan oder einer Titanlegierung und einer Beschichtung aus Iridium mit fraktaler Oberflächenstruktur (vgl. dazu Fig. 3), welche die Funktion hat, die aktive Oberfläche der Elektrodenabschnitte wesentlich zu vergrößern. Hierdurch wird vorteilhaft die Phasengrenzkapazität der Elektroden vergrößert und so die Phasengrenzimpedanz verringert. Zudem weisen die fraktalen Elektroden eine geringe Polarisierbarkeit auf, weshalb mit demselben Elektrodenpaar stimuliert und im wesentlichen artefaktfrei die Repolarisationsphase des stimulierten MAP gemessen werden kann. Elektroden mit fraktaler Beschichtung haben zudem in entsprechenden Untersuchungen der Erfinder einen geringen Einfluß des Elektrodenandrucks auf die MAP-Messung gezeigt, was in vorteilhafter Weise eine zur Vermeidung von Penetrationsgefahren geeignete Dimensionierung erlaubt.

Die Spitzenelektrode 1 weist mittig eine koaxial zur Symmetrieachse der Elektrodenanordnung verlaufende Bohrung zur Aufnahme einer Ag/AgCl-Elektrode 3 mit 0,3 mm Durchmesser auf, und zwischen den Elektroden 1 und 2a ist eine zweite Ag/AgCl-Elektrode 4 mit 0,3 mm Breite und 1,5 mm Länge angeordnet. Das Potential der Elektroden 1 und 3 wird durch das Potential des mit der Spize des Elektrodenkatheters kontaktierten Herzmuskels an der Kontaktstelle bestimmt und ist dadurch festgelegt, weshalb die Elektroden 1, 3 als differente Elektroden anzusprechen sind. Das aktuelle Potential der Elektroden 2a bis 2d und 4 ergibt sich hingegen aus dem zwischen den jeweils an die Meßanordnung angeschlossenen Elektroden fließenden Meßstrom, weshalb die Elektroden 2a bis 2d, 4 als indifferente Elektroden anzusprechen sind.

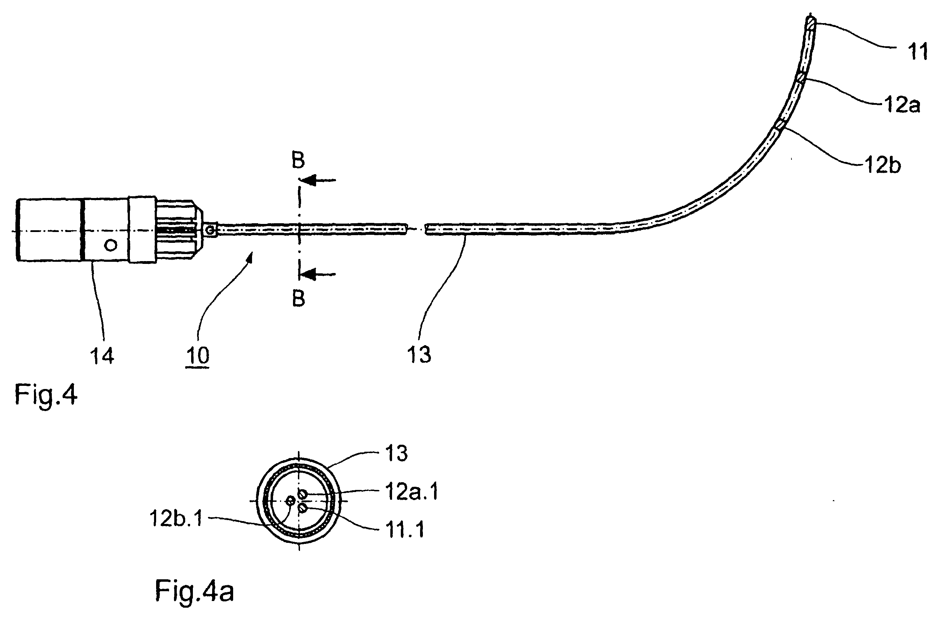

Die Elektrodenanordnung nach Fig. 2 ist speziell für vergleichende MAP-Messungen über fraktale Elektroden gegenüber der Erfassung über Ag/AgCl-Elektroden konzipiert, wie sie im Rahmen grundlegender Untersuchungen zweckmäßig ist. Bei der Ausführung als Mapping-Elektrodenkatheter für den Routineeinsatz können die Ag/AgCl-Elektroden 3 und 4 entfallen (vgl. Fig. 4 bis 6). Die Stelle der konzentrisch in die Spitzenelektrode 1 eingefügten Elektrode 3 kann bei einem kombinierten Mapping- und Ablationskatheter eine separate Ablationselektrode einnehmen.

Mit multipolaren Elektrodenkathetern in Art des in Fig. 2 gezeigten Aufbaus ist es möglich, mit nur einem Katheter an mehreren Stellen des Herzens gleichzeitig ein MAP-Signal abzuleiten, was völlig neue und zeitsparende Mapping-Strategien bei der Auffindung arrhythmogener Myokardareale - auch im Zusammenhang mit nicht-getriggerten Arrhythmien - ermöglicht. Des weiteren kann ein solcher Katheter sowohl zum Mapping als auch zugleich zur Ablation eingesetzt werden, womit zeit- und handhabungsaufwendige Katheterwechsel unnötig sind.

Die Fig. 3a bis 3c geben eine schematische Darstellung zur Verdeutlichung des schrittweisen Aufbaus der Oberflächenstruktur einer fraktalen Elektrode, wie sie beim erfindungsgemäßen Elektrodenkatheter - alternativ zu elektroaktiven Elektroden - vorgesehen ist. Zunächst erfolgt - wie in Fig. 3a zu erkennen - eine Ablagerung des auf eine glatte Unterlage (beispielsweise Titan bzw. eine Titanlegierung) abgeschiedenen Beschichtungsmaterials (etwa Iridium) in annähernd halbkugelförmigen, d.h. im Querschnitt halbkreisförmigen Aggregaten in dichter Packung. Die Grundform wird dann - wie Fig. 3b und 3c erkennen lassen - überlagert durch eine maßstäblich verkleinerte Wiederholung dieser Form in mehreren Stufen. Die verkleinerten Formelemente lagern sich dabei jeweils an der Oberfläche der nächst größeren Unterlage-Form an.

In der Praxis wird eine derartige fraktale Oberflächengeometrie beispielsweise durch geeignete Verfahrensführung eines elektrostatischen Sprühbeschichtungsverfahrens (Sputtern - speziell reaktive Kathodenzerstäubung) realisiert. Im Rahmen dieses kontinuierlichen Verfahrens sind die in den Figuren gezeigten Stufen natürlich zeitlich und räumlich nicht strikt zu trennen.) Als Beschichtungsmaterialien sind inerte - d.h. in Körperflüssigkeiten sehr geringe Oxidationsneigung aufweisende - Elemente, Legierungen oder Verbindungen geeignet, die eine fraktale Geometrie ausbilden können, beispielsweise grundsätzlich Elemente, Legierungen oder Nitride, Carbide oder Carbonitride aus der Gruppe Iridium, Platin, Gold oder auch Kohlenstoff.

Die starke und hochgradig differenzierte Tiefenstaffelung der nach hinreichender Prozeßdauer erhaltenen Struktur führt zu einer wesentlichen Vergrößerung der aktiven Elektrodenoberfläche um einen Faktor von bis zu 1000 oder mehr.

Die Figuren 4 und 4a zeigen eine (vereinfachte) Gesamtansicht bzw. eine Querschnittsdarstellung eines tripolaren steuerbaren Mapping-Elektrodenkatheters 10 mit einer Spitzenelektrode 11 sowie zwei von dieser beabstandeten Ringelektroden 12a und 12b auf einem langgestreckten, biegsamen. J-förmig vorgebogenen Trägerkörper 13, der am proximalen Ende eine an sich bekannte Betätigungs- und Anschlußvorrichtung 14 aufweist. Die Elektrode 11 hat eine Länge von 3,2 mm, die von der Spitzenelektrode bzw. voneinander um je 10 mm beabstandeten Ringelektroden 12a, 12b haben jeweils eine Länge von 2,2 mm. In dem in Fig. 4a gezeigten Querschnitt längs der Linie B-B in Fig. 4 ist zu erkennen, daß im hohlen Inneren des eine mehrschichtige Wandung aufweisenden Trägerkörpers 13 im wesentlichen parallel zueinander die drei Anschlußleitungen 11.1, 12a.1 und 12b.1 der Elektroden 11, 12a und 12b verlaufen.

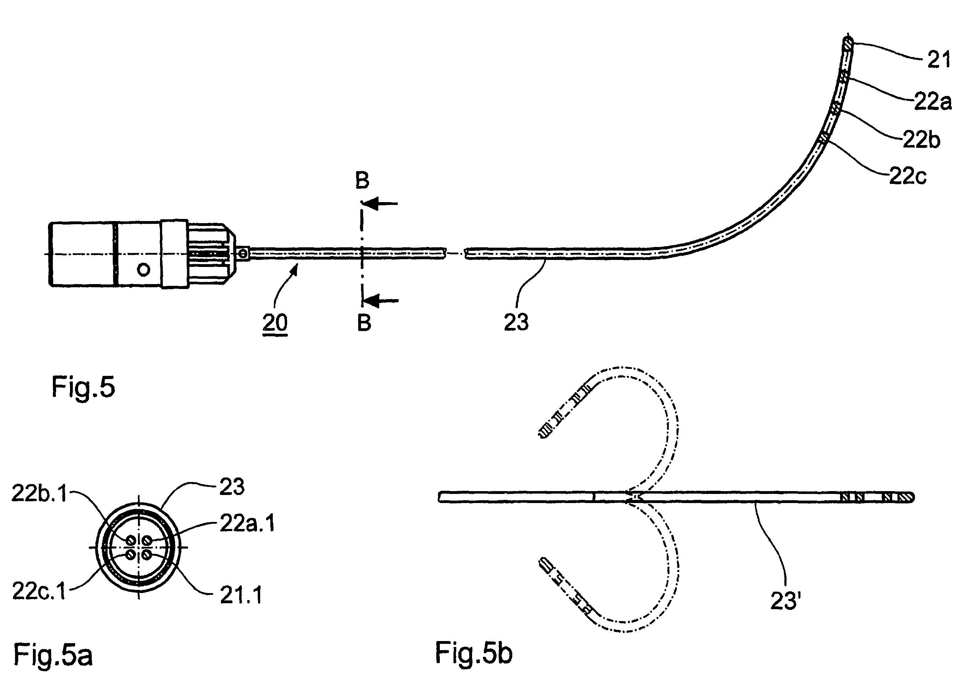

In ähnlicher Darstellung zeigen die Figuren 5 bis 5b eine vereinfachte Gesamtansicht sowie einen Querschnitt und eine zusätzliche Teilansicht eines (gemäß Fig. 5b in weitem Winkelbereich form-steuerbaren) Mapping-Elektrodenkatheters 20 in einer weiteren Ausführungsform. Der Trägerkörper 23 des Katheters 20 trägt eine quadrupolare Elektrodenanordnung, die eine Spitzenelektrode 21 (differente Elektrode) mit einer Länge von 3,2 mm und drei Ringelektroden (indifferente Elektroden) 22a, 22b und 22c von je 2,2 mm Länge und 5 mm Abstand zur jeweils benachbarten Elektrode umfaßt. Fig. 5a zeigt den Verlauf der Elektrodenzuleitungen 21.1, 22a.1, 22b.1 und 22c.1 im Trägerkörper 23.

Während der Elektrodenkatheter in Fig. 5 wiederum als in J-Form vorgebogener und damit zum Mapping für Standardanwendungen im Bereich des Atriums ausgebildeter Katheter gezeigt ist, zeigt Fig. 5b die Abwandlung eines Trägerkörpers 23' mit gerader Grundform, die - durch Vorsehen einer geeigneten, als solche bekannten mechanischen oder thermoelektrischen Steuereinrichtung mit Memory-Elementen im Inneren - in einem weiten Winkelbereich verformbar ist. Hiermit kann die Wandung des Atriums praktisch lückenlos kartiert werden, einschließlich des Mündungsbereiches der vena cava superior.

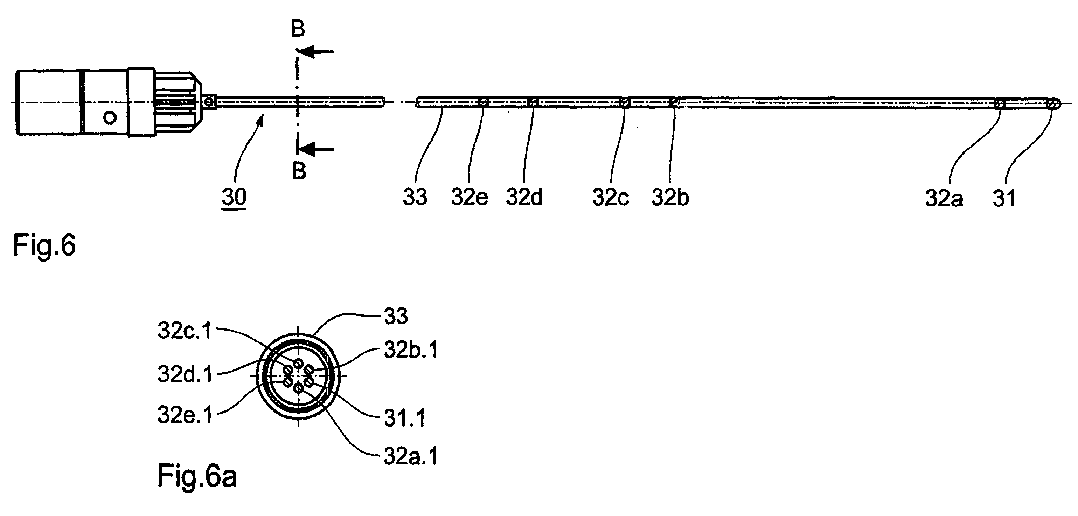

Die Figuren 6 und 6a sind eine (wiederum vereinfachte) Gesamtansicht bzw. eine Querschnittsdarstellung eines sechspoligen Mapping-Elektrodenkatheters 30 gemäß einer weiteren Ausführungsform. Während Aufbau und Abmessungen der Spitzenelektrode 31 und der Ringelektroden 32a bis 32e den vorgenannten Beispielen entsprechen, haben die Elektrodenabstände hier folgende Werte: d(31-32a) = 10 mm, d(32a-32b) = 80 mm, d(32b-32c) = 10 mm, d(32c-32d) = 20 mm, d(32d-32e) = 10 mm. Der in gestreckter Grundform gezeigte Trägerkörper 33 kann analog zu Fig. 5b wieder mit einer Steuerungseinrichtung zur Formsteuerung versehen sein.

Bei gleichzeitig zur Ablation eingesetzten Mapping-Kathetern ist zur möglichst treffsicheren Koagulation nur der als defekt ermittelten Herzgewebsbereiche eine starke Konzentration des wärmeenergieeintrags wünschenswert. In dieser Hinsicht ist bereits die Möglichkeit einer Reduzierung der makroskopischen Elektrodenabmessungen im Zusammenhnag mit der starken Vergrößerung der effektiven Oberfäche infolge der fraktalen Struktur bzw. der vorteilhaften Ausbillung als elektroaktive Oberfläche günstig. Weiter vorteilhaft sind eine (trotz minimierter Abmessungen) möglichst große Wärmekapazität und eine hohe Wärmeleitfähigkeit der Elektrode(n), die zur Übertragung der HF-Energie an das Gewebe ingesetzt wird/werden. Diesen Anforderungen wird in vorteilhafter Weiterbildung der Erfindung durch Ausführung dieser Elektrode(n) mit massiver Unterlage aus einem hoch wärmeleitfähigen Material Rechnung getragen. Des weiteren ist das Vorsehen einer speziellen Kühleinrichtung möglich.

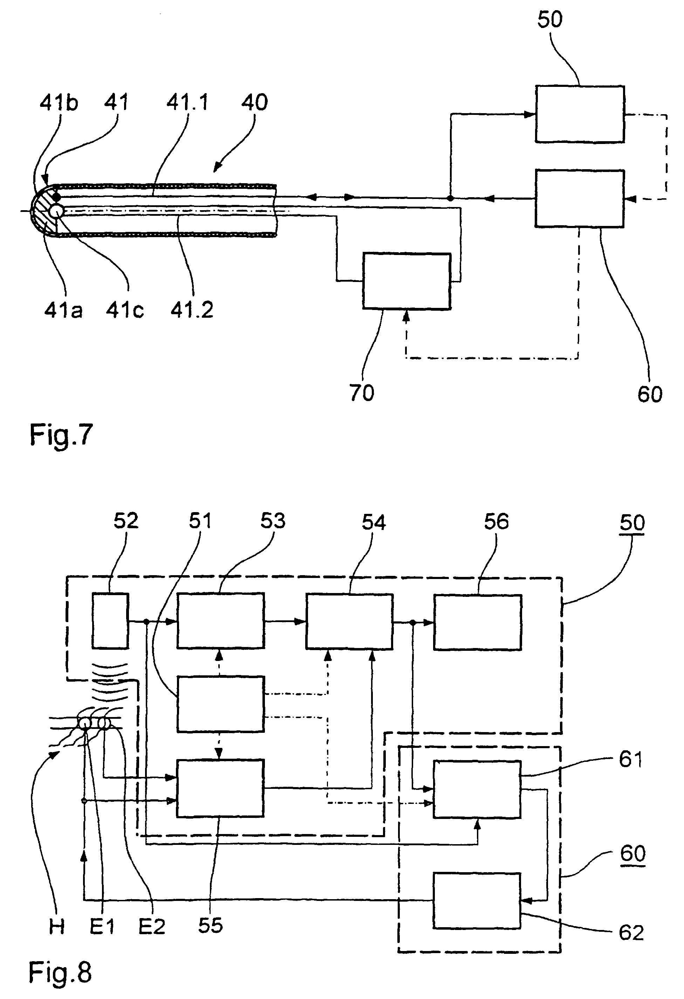

Figur 7 verdeutlicht in einer schematischen Darstellung das Grundprinzip des Aufbaus und Anschlusses der - dem Eintrag von Ablationsenergie dienenden - Spitzenelektrode 41 eines Mapping- und Ablationskatheters 40 gemäß einer weiteren Ausführungsform. Die Elektrode umfaßt einen massiven halbkugelförmigen Kern 41a aus einer Au-Ni-Legierung mit elektroaktiver Beschichtung 41b auf Iridiumoxidbasis und ein in den Kern eingefügtes Peltierelement 41c zur Kühlung. Die Elektrode 41 ist über eine Zuleitung 41.1 mit dem Eingang einer Erfassungs- und Verarbeitungsstufe 50 zur Bestimmung und Auswertung des MAP sowie zugleich mit dem Ausgang einer Ablationsstufe 60 verbunden, über die auf ein Steuersignal von der Stufe 50 hin der Elektrode ein Ablationsimpuls zugeführt wird. Zeitgleich oder kurz vor dessen Ausgabe wird zugleich eine über eine separate Zuleitung 41.2 mit dem Peltierelement 41c verbundene Kühlspannungsversorgung 70 angesteuert. Hierüber wird eine Kühlung der Elektrode 41 während der Ablation bewirkt.

Eine weitere zweckmäßige Weiterbildung des erfindungsgemäßen Elektrodenkatheters besteht im Vorsehen von, insbesondere aktiven, Fixierungsmitteln, beispielsweise einer (als solche bekannten) ausfahrbaren Schraubwendel an der Katheterspitze, mittels derer der Katheter beispielsweise nach der Ermittlung der optimalen Position für eine antitachykarde Stimulation anhand der Auswertung eines MAP-Mapping in dieser Position fixiert werden kann.

Figur 8 ist ein stark vereinfachtes Blockschaltbild einer Mapping-Anordnung unter Einsatz des erfindungsgemäßen Elektrodenkatheters, das hier symbolisch anhand zweier Elektroden E1 und E2 auf einem (nicht bezeichneten) Träger dargestellt ist. Die Elektroden des in das Atrium eines Herzens eingeführten Katheters befinden sich in Nachbarschaft zum atrialen Herzgewebe H. Ihre Position relativ zum Herzgewebe H wird unter Steuerung durch einen Controller 51 durch eine röntgenoptische Aufnahmevorrichtung 52 erfaßt, deren Ausgang mit einer Bildverarbeitungs- und Positionsermittlungseinrichtung 53 verbunden ist. In dieser werden die Raumkoordinaten der Elektrodenposition ermittelt und über deren Ausgang dem Adreßeingang einer Speichereinheit 54 zugeführt.

Gleichzeitig wird über die Elektroden E1, E2 ein - im dargestellten Beispiel ohne vorherige Stimulation erhaltenes, d.h. spontanes - Spannungssignal einer Vorhofaktion registriert und dem Eingang einer Signalverarbeitungseinheit 55 zugeführt, in der eine Signalaufbereitung, speziell Filterung mit niedriger Grenzfrequenz im Bereich von 0.3 bis 0,5 Hz, erfolgt. Am Ausgang der Stufe 55 steht ein lokales MAP-Signal bereit, welches in dem über die Positionsermittlungseinrichtung 53 adressierten Speicherplatz des Speichers 54 gespeichert wird. zugleich kann eine Anzeige über eine Anzeigeeinheit 56 erfolgen, mit der dem Arzt die erfolgte Speicherung signalisiert wird, woraufhin er den Katheter zur Fortsetzung der Kartierung neu positionieren kann. Auf diese Weise wird schrittweise die Wandung des Atriums abgetastet und das jeweilige lokale MAP in Zuordnung zu jeweiligen Elektrodenposition gespeichert, d.h. kartiert. Im Ergebnis läßt sich mit der Kartierungsvorrichtung 50 ein Bild der räumlichen Verteilung des MAP und hiermit arrhythmogener Myokardstrukturen erhalten und für den Arzt darstellen.

Mittels der Ablations-Steuereinrichtung 60 kann aufgrund der gespeicherten Kartierungsdaten eine automatische oder halbautomatische Steuerung des Katheters als Ablationskatheter vorgenommen werden. Hierzu werden die Speicherdaten einer Katheter-Steuereinrichtung 61 zugeführt, die - wie die übrigen Komponenten vom Controller 51 gesteuert - weiterhin zur Aufnahme und vergleichenden Verarbeitung von Bilddaten von der Aufnahmevorrichtung 52 ausgebildet ist und den Katheter einer vorbestimmten Position zusteuert. Ist eine in Auswertung des MAP einer Ablation zu unterziehende stelle des Herzgewebes H erreicht, wird ein Steuersignal an eine HF-Stromversorgung 62 ausgegeben und von dieser ein Ablations-Stromstoß der Elektrode E1 zugeführt.

Die Repositionierunmg des Katheters kann natürlich - unter Beobachtung über die Röntgenoptik 51 - auch manuell durch den Arzt erfolgen. Weiterhin kann eine Ablation ggfs. auch unmittelbar nach der Erfassung eines lokalen MAP an einer bestimmten Stelle des Herzgewebes allein aufgrund der Signalcharakteristik des lokalen Signals vorgenommen werden, so daß eine kartierende Speicherung und Repositionierung des Katheters nicht in jedem Anwendungsfall erforderlich ist. Weist der Katheter mehrere Elektrodenpaare auf, so schließt die Signalverarbeitung in einer Katheterstellung natürlich die Positionsbestimmung aller Erfassungs-Elektroden und die Erfassung und Speicherung sämtlicher von den einzelnen Elektrodenpaaren bei dieser Katheterstellung gelieferten Signale ein. Das Vorsehen einer Mehrzahl von Elektrodenpaaaren (wie bei den Kathetern nach Fig. 5 oder 6) verkürzt die MAP-Kartierung und ermöglicht ggfs. zusätzliche Aussagen zu bestimmten Korrelationen.

Insbesondere kann dabei alternierend oder auch - im wörtlichen Sinne - "gleichzeitig" über die Elektrode während der Hf-Ablation die MAP-Kenngröße gemessen werden. Auf diese Weise kann während der Hf-Ablation der Erfolg der Therapie beobachtet werden.

Der Begriff "Kartierung" schließt in den vorstehenden Ausführungen neben der räumlichen Verteilung auch jede Darstellung eines Signalparameters oder der Signalmorphologie ein. Die Zahl der dazu benötigten Elektroden ist je nach Geometrie und abzuleitender Größe (geometrisch/flächenbezogen) oder einfacher Signalkennwert zwei oder drei.

## Patentansprüche

1. Elektrodenkatheter zur kartierenden Erfassung und lokalisierten Zerstörung von Reizleitungsstrukturen im Herzgewebe mit mindestens zwei gegeneinander isolierten Elektroden (E1, E2) zur kartierenden Erfassung eines Herzaktionspotentials, insbesondere des atrialen monophasischen Aktionspotentials (MAP), wobei die Elektroden (E1,E2) eine massive, metallische Unterlage mit hoher Leitfähigkeit und darauf eine Beschichtung mit geringer Polarisierbarkeit aufweisen, die elektroaktiv ist oder eine fraktale Oberflächengeometrie besitzt, und wobei mindestens eine Elektrode (E1) mit dem Eingang einer Erfassungs- und Verarbeitungsstufe (50) sowie zugleich mit dem Ausgang einer Ablationsstufe (60) verbunden ist.

2. Elektrodenkatheter nach Anspruch 1, **dadurch gekennzeichnet, dass** die Elektroden (E1,E2) eine im wesentlichen aus Iridiumoxid bestehende elektroaktive Beschichtung aufweisen.

3. Elektrodenkatheter nach Anspruch 1 oder 2, **dadurch gekennzeichnet, dass** die Elektroden (E1,E2) eine im wesentlichen aus Iridium oder Iridiumnitrid bestehende Beschichtung mit fraktaler Oberflächengeometrie aufweisen.

4. Elektrodenkatheter nach einem der vorangehenden Ansprüche, **dadurch gekennzeichnet, dass** zumindest zwei Elektroden (E1, E2) vorgesehen sind, die ein Paar bipolar angeschlossener Erfassungs-Elektroden bilden.

5. Elektrodenkatheter nach Anspruch 4, **dadurch gekennzeichnet, dass** jeweils mindestens ein Paar ring- oder haibringförmige Erfassungs-Elektroden auf einem langgestreckten flexiblen Träger angeordnet sind.

6. Elektrodenkatheter nach einem der vorangehenden Ansprüche, **dadurch gekennzeichnet, dass** alle Elektroden auf einem einzelnen langgestreckten flexiblen Träger angeordnet sind.

7. Elektrodenkatheter nach Anspruch 5 oder 6, **dadurch gekennzeichnet, dass** dem Träger eine Steuerungseinrichtung zur Einstellung einer vorgegebenen gekrümmten Gestalt zugeordnet ist.

8. Elektrodenkatheter nach einem der vorangehenden Ansprüche, **gekennzeichnet durch** eine aktive räumlich mindestens einer der Elektroden (E1, E2) zugeordnete Kühleinrichtung.

9. Elektrodenkatheter nach einem der vorangehenden Ansprüche, **gekennzeichnet durch** steuerbare Mittel zur Fixierung der Position des distalen Endes des Elektrodenkatheters relativ zum Herzgewebe.

10. Anordnung zur kartierenden Erfassung und lokalisierten Zerstörung von Reizleitungsstrukturen im Herzgewebe, mit
(a) einem Elektrodenkatheter mit mindestens zwei gegeneinander isolierten Elektroden (E1, E2) zur kartierenden Erfassung eines Herzaktionspotentials, insbesondere des atrialen monophasischen Aktionspotentials (MAP), wobei die Elektroden (E1,E2) eine massive, metallische Unterlage mit hoher Leitfähigkeit und darauf einer Beschichtung mit geringer Polarisierbarkeit aufweisen, die elektroaktiv ist oder eine fraktale Oberflächengeometrie besitzt, und wobei mindestens eine Elektrode (E1) mit dem Eingang einer Erfassungs- und Verarbeitungsstufe (50) sowie zugleich mit dem Ausgang einer Ablationsstufe (60) verbunden ist, und wobei die zumindest zwei Elektroden (E1, E2) ein Paar bipolar angeschlossener Erfassungs-Elektroden bilden,
(b) einer separat mit den Erfassungs-Elektroden verbundenen Herzsignal-Verarbeitungseinrichtung (55) zur lokalen Ermittlung eines Herzaktionspotentials an den Orten der Elektroden,
(c) einer Positionsermittlungseinrichtung (53) zur Ermittlung der räumlichen Lage der Erfassungs-Elektroden relativ zum Herzgewebe,
(d) einer mit den Ausgängen der Verarbeitungseinrichtung und der Positionsermittlungseinrichtung verbundenen Speichereinrichtung (54) zur Speicherung der lokalen Herzaktionspotentiale in Zuordnung zu den zugehörigen Elektrodenorten und
(e) einer eingangsseitig mit der Speichereinrichtung verbundene Steuereinheit (61) zur Positionierung des Elektrodenkatheters und zur Steuerung der Zuführung von Ablationsenergie zu mindestens einer der Elektroden (E1), die mit dem Ausgang der Ablationsstufe (60) verbunden ist, in Abhängigkeit von den lokalen Herzaktionspotentialen.

11. Anordnung nach Anspruch 10, **dadurch gekennzeichnet, dass** eine Anzeigeeinheit (56) zur Darstellung der räumlichen Verteilung des Herzaktionspotentials über das Herzgewebe bzw. einer entsprechenden Kenngröße vorgesehen ist.

## Claims

1. Electrode catheter for detection by mapping and localised destruction of heart conduction structures in the heart tissue with at least two electrodes (E1, E2), insulated against one another, for detection by mapping of a heart action potential, in particular of the atrial monophase action potential (MAP), the electrodes (E1, E2) having a solid metal base with high conductivity and on this a coating with low polarisability, which is electroactive or has a fractal surface geometry, and at least one electrode (E1) being connected to the input of a detection and processing step (50) and also simultaneously to the output of an ablation slap (60).

2. Electrode catheter according to claim 1, **characterised in that** the electrodes (E1, E2) have an electroactive coating consisting substantially of iridium oxide.

3. Electrode catheter according to claim 1 or 2, **characterised in that** the electrodes (E1, E2) have a coating consisting substantially of iridium or iridium nitride with fractal surface geometry.

4. Electrode catheter according to one of the preceding claims, **characterised in that** at least two electrodes (E1, E2) are provided, forming a pair of detection electrodes connected in bipolar manner.

5. Electrode catheter according to claim 4, **characterised in that** in each case at least one pair of annular or semi-annular detection electrodes is arranged on an elongated flexible carrier.

6. Electrode catheter according to one of the preceding claims, **characterised in that** all the electrodes are arranged on a single elongated flexible carrier.

7. Electrode catheter according to claim 5 or 6, **characterised in that** assigned to the carrier is a control device for setting a preset curved shape.

8. Electrode catheter according to one of the preceding claims, **characterised by** an active cooling device assigned spatially to at least one of the electrodes (E1, E2).

9. Electrode catheter according to one of the preceding claims, **characterised by** controllable means for fixing the position of the distal end of the electrode catheter relative to the heart tissue.

10. Arrangement for detection by mapping and localised destruction of heart conduction structures in the heart tissue, with
a) an electrode catheter with at least two electrodes (E1, E2), insulated against one another, for detection by mapping of a heart action potential, in particular of the atrial monophase action potential (MAP), the electrodes (E1, E2) having a solid metal base with high conductivity and on this a coating with low polarisability, which is electroactive or has a fractal surface geometry, and at least one electrode (E1) being connected to the input of a detection and processing step (50) and also simultaneously to the output of an ablation step (60) and the at least two electrodes (E1, E2) forming a pair of detection electrodes connected in bipolar manner,
(b) a heart signal processing device (55), connected separately to the detection electrodes, for local determination of a heart action potential at the sites of the electrodes,
(c) a position determining device (53) for determining the spatial position of the detection electrodes relative to the heart tissue,
(d) a memory device (54) connected to the outputs of the processing device and the position determining device for storing the local heart action potentials assigned to the associated electrode sites and
(e) a control unit (61), connected on the input side to the memory device, for positioning the electrode catheter and for controlling the feed of ablation energy to at least one of the electrodes (E1), which is connected to the output of the ablation step (60), as a function of the local heart action potentials.

11. Arrangement according to claim 10, **characterised in that** a display unit (56) for illustrating the spatial distribution of the heart action potential over the heart tissue or a corresponding parameter is provided.

## Revendications

1. Cathéter à électrodes pour la détection de cartographie et la destruction localisée de structures de conduction stimulatrice dans le tissu cardiaque comportant au moins deux électrodes isolées l'une par rapport à l'autre (E1, E2) pour la détection de cartographie d'un potentiel d'action cardiaque, en particulier du potentiel d'action monophasique auriculaire (MAP), où les électrodes (E1, E2) comportent une base métallique massive à haute conductibilité et, sur celle-ci, un revêtement à faible polarisabilité qui est électroactif ou qui possède une géométrie superficielle fractale, et où au moins une électrode (E1) est reliée à l'entrée d'un étage de détection et de traitement (50) et simultanément à la sortie d'un étage d'ablation (60).

2. Cathéter à électrodes selon la revendication 1 **caractérisé en ce que** les électrodes (E1, E2) comportent un revêtement électroactif consistant essentiellement en oxyde d'iridium.

3. Cathéter à électrodes selon la revendication 1 ou 2 **caractérisé en ce que** les électrodes (E1, E2) comportent un revêtement consistant essentiellement en iridium ou en nitrure d'iridium à géométrie superficielle fractale.

4. Cathéter à électrodes selon l'une des revendications précédentes **caractérisé en ce qu'**il est prévu au moins deux électrodes (E1, E2) qui forment une paire d'électrodes de détection raccordées de manière bipolaire.

5. Cathéter à électrodes selon la revendication 4 **caractérisé en ce que**, dans chaque cas, au moins une paire d'électrodes de détection en forme d'anneau ou de demi-anneau est disposée sur un support flexible allongé.

6. Cathéter à électrodes selon l'une des revendications précédentes **caractérisé en ce que** toutes les électrodes sont disposées sur un support flexible allongé unique.

7. Cathéter à électrodes selon la revendication 5 ou 6 **caractérisé en ce qu'**un dispositif de commande pour établir une configuration courbée prédéterminée est associé au support.

8. Cathéter à électrodes selon l'une des revendications précédentes **caractérisé par** un dispositif réfrigérant actif associé spatialement à au moins l'une des électrodes (E1, E2).

9. Cathéter à électrodes selon l'une des revendications précédentes **caractérisé par** des moyens pouvant être commandés pour fixer la position de l'extrémité distale du cathéter à électrodes par rapport au tissu cardiaque.

10. Agencement pour la détection de cartographie et la destruction localisée de structures de conduction stimulatrice dans le tissu cardiaque, avec
(a) un cathéter à électrodes comportant au moins deux électrodes isolées l'une par rapport à l'autre (E1, E2) pour la détection de cartographie d'un potentiel d'action cardiaque, en particulier du potentiel d'action monophasique auriculaire (MAP), où les électrodes (E1, E2) comportent une base métallique massive à haute conductibilité et, sur celle-ci, un revêtement à faible polarisabilité qui est électroactif ou qui possède une géométrie superficielle fractale, et où au moins une électrode (E1) est reliée à l'entrée d'un étage de détection et de traitement (50) et simultanément à la sortie d'un étage d'ablation (60), et où les électrodes (E1, E2) au nombre de deux au moins forment une paire d'électrodes de détection raccordées de manière bipolaire,
(b) un dispositif de traitement des signaux cardiaques (55) relié séparément aux électrodes de détection pour la détermination locale d'un potentiel d'action cardiaque aux sites des électrodes,
(c) un dispositif de détermination de position (53) pour déterminer la position spatiale des électrodes de détection par rapport au tissu cardiaque,
(d) un dispositif de mémorisation (54) relié aux sorties du dispositif de traitement et du dispositif de détermination de position pour la mémorisation des potentiels d'action cardiaques locaux en correspondance avec les sites d'électrodes associés et
(e) une unité de commande (61) reliée du côté de l'entrée au dispositif de mémorisation pour le positionnement du cathéter à électrodes et pour la commande de l'apport d'énergie d'ablation à au moins l'une des électrodes (E1) qui est reliée à la sortie de l'étage d'ablation (60), en fonction des potentiels d'action cardiaques locaux.

11. Agencement selon la revendication 10 **caractérisé en ce qu'**il est prévu une unité indicatrice (56) pour représenter la répartition spatiale du potentiel d'action cardiaque sur le tissu cardiaque ou d'un paramètre correspondant.
